# EUROPÄISCHE PATENTSCHRIFT

(11) **EP 0 729 954 B1**
(45) Veröffentlichungstag und Bekanntmachung des Hinweises auf die Patenterteilung: **03.09.2003**
(21) Anmeldenummer: 96101096.4
(22) Anmeldetag: 26.01.1996
(51) Int. Cl.: C07D 333/34, A61K 31/64

(54) **Substituierte Thiophenylsulfonylharnstoffe und -thioharnstoffe, Verfahren zu ihrer Herstellung, ihre Verwendung als Medikament oder Diagnostikum sowie sie enthaltendes Medikament**
Substituted thiophene sulfonyl ureas and thioureas, process for their preparation, their use as medicaments
Thiophénylurées et -thiourées, procédé pour leur préparation et leur utilisation comme médicament

(30) Priorität: 01.02.1995 DE 19503136
(43) Veröffentlichungstag der Anmeldung: 04.09.1996
(73) Patentinhaber: HOECHST AKTIENGESELLSCHAFT, 65929 Frankfurt am Main (DE)
(72) Erfinder: Englert, Heinrich, Dr., D-65719 Hofheim (DE); Hartung, Jens, Dr., D-97204 Höchberg (DE); Crause, Peter, Dr., D-63069 Offenbach (DE); Mania, Dieter, Dr., D-61462 Königstein (DE); Gögelein, Heinz, Dr., D-60259 Frankfurt (DE); Kaiser, Joachim, Dr., D-60431 Frankfurt (DE)

(56) Entgegenhaltungen:
- EP-A- 0 612 724
- EP-A- 0 657 423
- EP-A- 0 661 264
- DE-A- 1 518 874
- DE-A- 1 940 131
- DE-A- 2 413 514
- FR-A- 2 068 472
- SCIENCE, Bd. 247, Februar 1990, WASHINGTON, Seiten 852-854, XP002003650 AMOROSO,S. ET AL.: "Glucose, Sulfonylureas, and Neurotransmitter Release : Role of ATP-sensitive K+ Channels"
- THE LANCET, Bd. 8453, 31.August 1985, LONDON, Seiten 474-475, XP002003651 STURGESS,N. ET AL.: "The Sulphonylurea Receptor may be an ATP-sensitive Potassium Channel"
- JORN.ANNU.DIABETOL.HOTEL-DIEU, 1989, Seiten 7-11, XP002003652 FOSSET,M. ET AL.: "Identification, mécanismes de fonctionnement et régulation des canaux potassium sensibles à l'ATP cibles des sulfonylurées utilisées dans le traitement du diabète de type II "

## Beschreibung

Substituierte Thiophenylsulfonylharnstoffe und -thioharnstoffe, Verfahren zu ihrer Herstellung, ihre Verwendung als Medikament oder Diagnostikum sowie sie enthaltendes Medikament

Die Erfindung betrifft substituierte Thiophensulfonylharnstoffe und -thioharnstoffe I 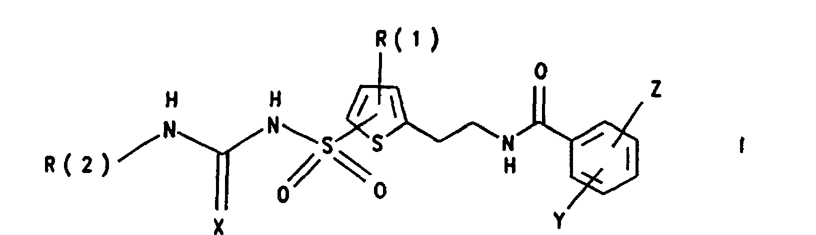 in denen bedeuten:
- R(1): Wasserstoff oder Methyl;
- R(2): Wasserstoff oder Methyl;
- X: Sauerstoff (Verbindungen I a) oder Schwefel (Verbindungen I b);
- Y, Z: voneinander verschieden Fluor, Chlor, Methoxy oder Ethoxy;
und ihre physiologisch unbedenklichen Salze.

Ähnliche Thiophensulfonylharnstoffe sind aus der Niederländischen Patentanmeldung NL 70 11 219 bekannt; ähnliche Benzolsulfonylharnstoffe sind aus den Deutschen Offenlegungsschriften 2 413 514 und 1 518 874 bekannt. Dort wird deren blutzuckersenkende Wirkung beschrieben. Als Prototyp solcher blutzuckersenkenden Sulfonylharnstoffe gilt das Glibenclamid, das als Mittel zur Behandlung des Diabetes mellitus therapeutisch verwendet wird und in der Forschung ein vielbeachtetes Werkzeug zur Erforschung sogenannter ATP-sensitiver Kaliumkanäle dient. Neben seiner Blutzucker-senkenden Wirkung besitzt das Glibenclamid noch andere Wirkungen, die bislang therapeutisch noch nicht eingesetzt werden können, die aber allesamt auf Blockade eben dieser ATP-sensitiven Kalium-Kanäle zurückgeführt werden. Dazu gehört insbesondere eine antifibrillatorische Wirkung am Herzen. Bei der Behandlung des Kammerflimmerns oder seiner Vorstufen wäre jedoch eine gleichzeitige Blutzuckersenkung unerwünscht oder gar gefährlich da sie den Zustand des Patienten weiter verschlechtern kann.

Aufgabe der vorliegenden Erfindung war es daher, Verbindungen zu synthetisieren, die eine gleich gute Herzwirkung wie Glibenclamid aufweisen, aber den Blutzucker in herzwirksamen Dosen oder Konzentrationen nicht oder deutlich geringer beeinflussen als Glibenclamid. Sulfonylhamstoffe und Thioharnstoffe mit bevorzugter Wirkung am Herzen sind schon aus der Europäischen Offenlegungsschrift 0 612 724 bekannt.

Eine Ausführungsform der Erfindung betrifft Verbindungen I, in denen bedeuten:
- R(1): Wasserstoff oder Methyl;
- R(2): Wasserstoff oder Methyl;
- X: Sauerstoff;
- Z, Y: voneinander verschieden, Fluor, Chlor, Methoxy oder Ethoxy;
und ihre physiologisch unbedenklichen Salze.

Eine andere Ausführungsform der Erfindung betrifft Verbindungen I, in denen bedeuten:
- R(1): Wasserstoff oder Methyl;
- R(2): Wasserstoff oder Methyl;
- X: Schwefel;
- Z, Y: voneinander verschieden, Fluor, Chlor, Methoxy oder Ethoxy;
und ihre physiologisch unbedenklichen Salze.

Die Verbindungen der vorliegenden Erfindung sind wertvolle Arzneimittel zur Behandlung von Herzrhythmusstörungen unterschiedlichster Genese und zur Verhinderung des arrhythmisch bedingten, plötzlichen Herztodes und können daher als Antiarrhythmika Verwendung finden. Beispiele arrhythmischer Störungen des Herzens sind supraventrikuläre Rhythmusstörungen wie etwa Vorhof-Tachycardien, Vorhof-Flattern oder paroxysmale supraventriculäre Rhythmusstörungen oder ventrikuläre Rhythmusstörungen wie ventrikuläre Extrasystolen insbesondere aber lebensbedrohende ventrikuläre Tachykardien oder das besonders gefährliche Kammerflimmem. Sie eignen sich insbesondere für solche Fälle wo Arrhythmien Folge einer Verengung eines Koronargefäßes sind, wie sie beispielsweise bei Angina Pectoris oder während eines akuten Herzinfarktes oder als chronische Folge eines Herzinfarktes auftreten. Sie sind daher insbesondere bei Postinfarktpatienten zur Verhinderung des plötzlichen Herztodes geeignet. Weitere Krankheitsbilder, bei denen derartige Rhythmusstörungen und/oder der plötzliche, arrhythmisch bedingte Herztod eine Rolle spielen, sind beispielsweise die Herzinsuffizienz oder die Herzhypertrophie als Folge eines chronisch erhöhten Blutdrucks.

Darüberhinaus können die Verbindungen eine verminderte Kontraktilität des Herzens positiv beeinflussen. Hierbei kann es sich um ein krankheitsbedingtes Nachlassen der Herzkontraktilität handeln wie beispielsweise bei Herzinsuffizienz aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Stillegung der Herzaktivität durch cardioplegische Lösungen erforderlich machen.

Die Erfindung betrifft weiterhin ein Verfahren zur Herstellung der Verbindungen I, das dadurch gekennzeichnet ist, daß man
(a) aromatische Sulfonamide der Formel II 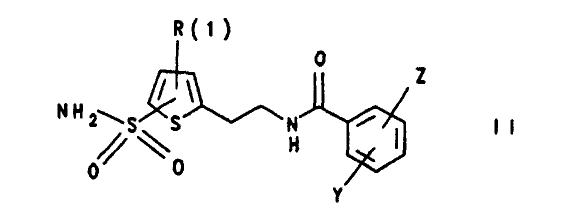 oder deren Salze der Formel III 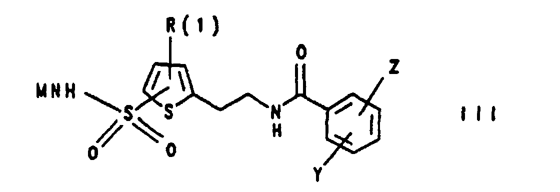 mit R(2)-substituierten Isocyanaten der Formel IV

   R(2)-N=C=O IV

   zu substituierten Thiophensulfonylharnstoffen I umsetzt.
   Als Kationen M in den Salzen der Formel II kommen Alkali-, Erdalkali-, Ammonium- sowie Tetraalkylammonium-lonen in Betracht. Äquivalent zu den R(2)-substituierten Isocyanaten IV kann man R(2)-substituierte Carbamidsäureester, R(2)-substituierte Carbamidsäurehalogenide oder R(2)-substituierte Harnstoffe einsetzen;
(b) Unsubstituierte Thiophensulfonylharnstoffe I a [R(2) = H] 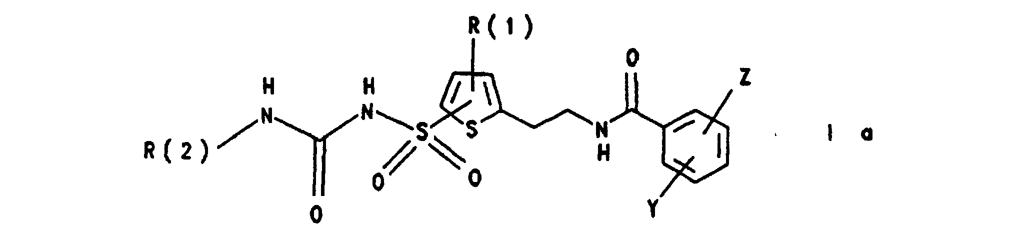 kann man durch Umsetzungen aromatischer Thiophensulfonamide der Formel II oder deren Salze III mit Trialkylsilylisocyanat oder Siliciumtetraisocyanat und Spaltung (z. B. Hydrolyse) der primären Silicium-substituierten Thiophensulfonylharnstoffe herstellen. Weiterhin ist es möglich, Thiophensulfonamide 2 oder deren Salze III durch Umsetzung mit Halogencyanen und Hydrolyse der primär entstehenden N-Cyanosulfonamide mit Mineralsäuren bei Temperaturen von 0°C bis 100°C darzustellen.
(c) Thiophensulfonylharnstoffe I a lassen sich aus aromatischen Thiophensulfonamiden II oder deren Salze III aus R(2)-substituierten Trichloracetamiden der Formel V 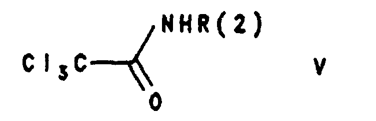
in Gegenwart einer Base in einem inerten Lösungsmittel nach Synthesis 1987, 734 - 735 bei Temperaturen von 25°C bis 150°C darstellen.

Als Basen eignen sich zum Beispiel Alkalimetall- oder Erdalkalimetallhydroxide, -hydride, -amide oder auch -alkoholate, wie Natriumhydroxid, Kaliumhydroxid, Calciumhydroxyd, Natriumhydrid, Kaliumhydrid, Calciumhydrid, Natriumamid, Kaliumamid, Natriummethylat, Natriumethanolat, Kaliummethylat oder Kaliumethanolat.
Als inerte Lösungsmittel eignen sich Ether wie Tetrahydrofuran, Dioxan, Ethylenglykoldimethylether (Diglyme), Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.

### (d) Thiophensulfonylthioharnstoffe I b

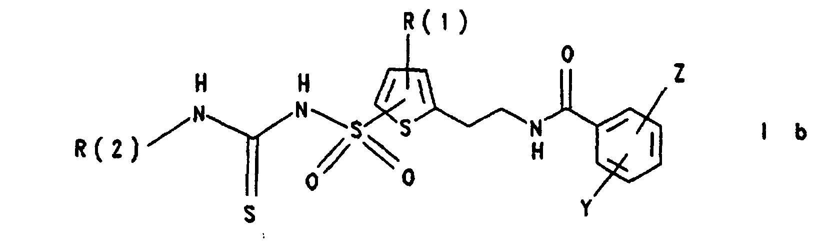

werden aus Thiophensulfonamiden II sowie deren Salze III und R(2)-substituierten Isothiocyanaten VI

R(2)-N=C=S VI

dargestellt. Unsubstituierte Thiophensulfonylthioharnstoffe I [R(2) = H] kann man durch Umsetzungen aromatischer Thiophensulfonamide II oder von deren Salzen III mit Trimethylsilylisothiocyanat oder Siliciumtetraisothiocynat und Spaltung (Hydrolyse) der primär entstehenden Silicium-substituierten Thiophensulfonylharnstoffe herstellen. Weiterhin ist es möglich, aromatische Thiophensulfonamide II oder deren Salze II mit Benzoylisothiocyanat umzusetzen und die intermediären benzoylsubstituierten Thiophensulfonylthioharnstoffe mit wäßrigen Mineralsäuren zu I b [R(2) = H] umzusetzen. Ähnliche Verfahren sind beschrieben in J. Med. Chem. 1992, 35, 1137 - 1144. Eine weitere Variante besteht darin, die unter Verfahren a erwähnten N-Cyansulfonamide mit Schwefelwasserstoff umzusetzen.
e) Substituierte Thiophensulfonylharnstoffe der Formel I a können durch Umwandlungsreaktionen von Thiophensulfonylthioharnstoffen der Struktur I b dargestellt werden. Der Ersatz des Schwefelatoms durch ein Sauerstoffatom in den entsprechend substituierten Thiophensulfonylthioharnstoffen kann beispielsweise mit Hilfe von Oxiden oder Salzen von Schwermetallen oder auch durch Anwendung von Oxidationsmitteln (wie Wasserstoffperoxid, Natriumperoxid oder salpetriger Säure) ausgeführt werden. Thioharnstoffe können auch durch Behandlung mit Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Thiophensulfonylharnstoffe überführt werden. Isothioharnstoffe verhalten sich bei der Entschwefelung wie Thioharnstoffe und können demzufolge ebenso als Ausgangsstoffe für diese Reaktionen dienen.
(f) Thiophensulfonylharnstoffe I a können aus Thiophensulfonylhalogeniden der Formel VII 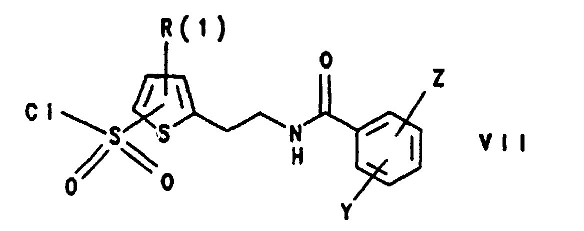 mit R(2)-substituierten Harnstoffen oder R(2)-substituierten Bis(trialkylsilyl)harnstoffen hergestellt werden. Die Trialkylsilylschutzgruppe kann aus dem resultierenden (Trialkylsilyl)-thiophensulfonylharnstoff nach Standardmethoden entfernt werden. Weiterhin kann man die Sulfonsäurechloride VII mit Parabansäuren zu Thiophensulfonylparabansäuren umsetzen, deren Hydrolyse mit Mineralsäuren die entsprechenden Thiophensulfonylharnstoffe I a liefert.
(g) Thiophensulfonylharnstoffe I a lassen sich durch Umsetzungen von Aminen der Formel R(2)-NH₂ mit Thiophensulfonylisocyanaten der Formel VIII 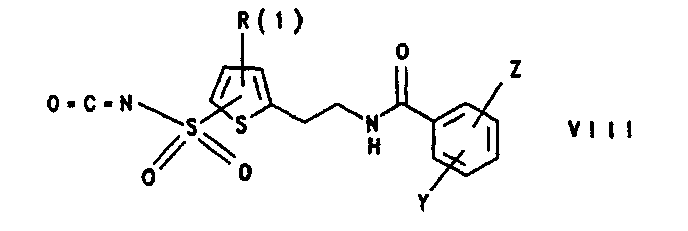 herstellen. Ebenso können Amine R(2)-NH₂ mit Thiophensulfonylcarbamidsäureester, -carbamidsäurehalogenide oder Thiophensulfonyl-harnstoffe I a [mit R(2) = H] zu den Verbindungen I a umgesetzt werden.
(h) Thiophensulfonylthioharnstoffe I b lassen sich durch Umsetzungen von Aminen der Formel R(2)-NH₂ mit Thiophensulfonylisothiocyanaten der Formel IX 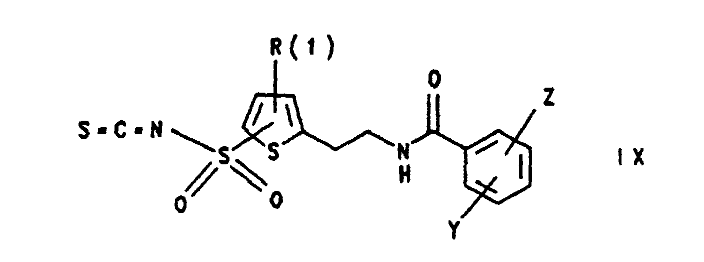 herstellen. Ebenso können Amine R(1)-NH₂ mit Thiophensulfonylcarbamidsäurethioestern oder -carbamidsäurethiohalogeniden zu den Verbindungen I b umgesetzt werden.
(i) Entsprechend substituierte Benzolsulfenyl- oder -sulfinylharnstoffe lassen sich mit Oxidationsmitteln wie Wasserstoffperoxid, Natriumperoxid oder salpetriger Säure zu Thiophensulfonylharnstoffen I a oxidieren.

Verbindungen I sowie deren physiologisch unbedenkliche Salze sind wertvolle Therapeutika, die sich nicht nur als Antiarrhythmika, sondern auch zur Prophylaxe bei Störungen des cardiovaskulären Systems, bei der Herzinsuffizienz, bei Herztransplantationen oder cerebralen Gefäßerkrankungen an Menschen oder Säugetieren (zum Beispiel Affen, Hunde, Mäuse, Ratten, Kaninchen, Meerschweinchen und Katzen) eignen.

Unter physiologisch unbedenklichen Salzen der Verbindungen I versteht man nach Remmington's Pharmaceutical Science, 17. Auflage, 1985, Seiten 14 - 18 Verbindungen der Formel X, 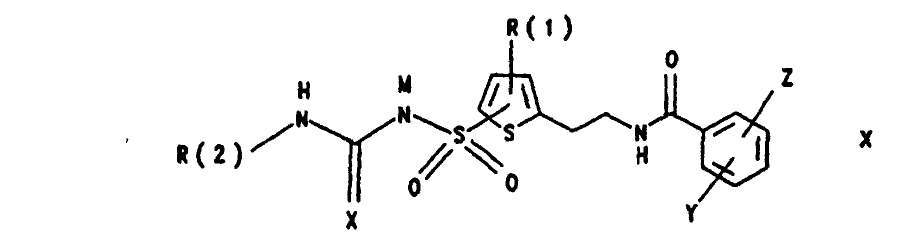 die sich aus nicht toxischen organischen und anorganischen Basen und substituierten Thiophensulfonylharnstoffen I darstellen lassen. Bevorzugt werden hierbei Salze, in denen M in der Formel X Natrium-, Kalium-, Rubidium-, Calcium-, Magnesium-, Ammoniumionen ist, sowie die Säureadditionsprodukte basischer Aminosäuren, wie Lysin oder Arginin sein können.

Die Ausgangsverbindungen für die erwähnten Syntheseverfahren der Thiophensulfonylharnstoffe werden nach bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

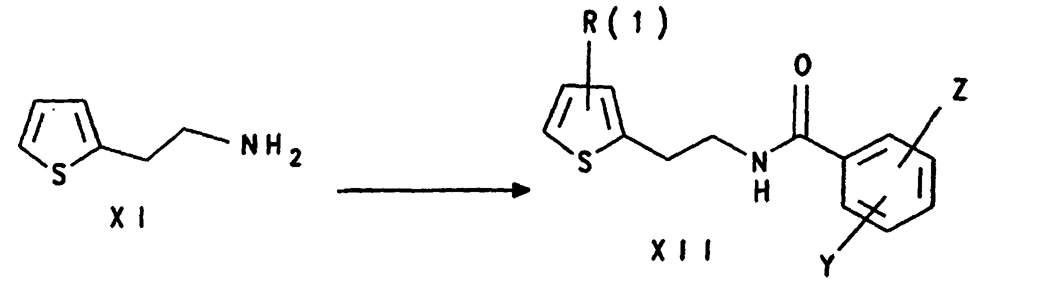

### Schema 1

So kann man geeignet substituierte Amine der Formel XI nach Schema 1 acylieren und einer Halogensulfonierung unterwerfen. Als acylierende Mittel zur Acylierung von Aminogruppen eignen sich zweckmäßig die Alkylester, Halogenide (zum Beispiel Chloride oder Bromide) oder Anhydride von Carbonsäuren der Formel R₄-COB. R₄ steht hierbei für Benzoesäurederivat. Das Benzoesäurederivat ist hierbei durch zwei verschiedene Reste Y, Z in der eingangs definierten Bedeutung substituiert. B ist eine Fluchtgruppe wie zum Beispiel Halogenid, Alkoxy mit 1, 2, 3 oder 4 C-Atomen, Trihalogenacetat, (C₁-C₄)-Carboxylat. Beispiele hierfür sind 5-Chlor-2-methoxybenzoesäurechlorid oder -anhydrid sowie -(C₁-C₄)-alkylester. Die Synthesen der Verbindung XII werden unter Zusatz einer tertiären Base (wie von Pyridin oder einem Trialkylamin) in Gegenwart oder Abwesenheit eines inerten Lösungsmittels durchgeführt, wobei auch ein Katalysator, wie Dimethylaminopyridin, zugegen sein kann. Die Umsetzung kann bei Temperaturen von etwa 0°C bis 160°C, vorzugsweise von 20 bis 150°C, erreicht werden. Als inerte Lösungsmittel eignen sich Ether (wie Tetrahydrofuran, Dioxan), Glykolether wie Ethylenglykolmonomethyl- oder monoethylether (Methylglykol oder Ethylglykol), Ethylenglykoldimethylether (Diglyme), Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Phosphorsäurehexamethyltriamid, Sulfoxide wie DMSO, chlorierte Kohlenwasserstoffe wie Dichlormethan, Chloroform, Trichlorethylen, 1,2-Dichlorethan oder Kohlenstofftetrachlorid, Kohlenwasserstoffe wie Benzol, Toluol, Xylole. Weiterhin eignen sich auch Gemische dieser Lösungsmittel untereinander.
Amine XI sind im allgemeinen durch Standardverfahren erhältlich, beispielsweise aus Thiophen-2-carbaldehyden, die mit Nitromethan zu den entsprechenden Nitroolefinen umgesetzt werden, und dann einer Reduktion unterworfen werden, beispielsweise mit LiAlH₄, und dabei XI ergeben.

Die nach Schema 1 acylierten Amine XII können in bekannter Weise nach Schema 2 in die Sulfonamide XIII überführt werden: 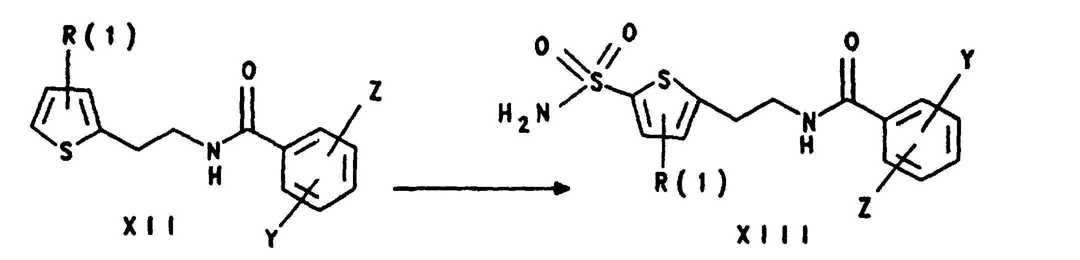 Die Sulfonamide XIII werden nach bekannten Methoden hergestellt und zwar unter Reaktionsbedingungen, die für die genannten Umsetzungen bekannt und geeignet sind. Dabei kann man auch von bekannten, hier aber nicht näher erwähnten Varianten Gebrauch machen. Die Synthesen können, falls gewünscht, in einem, zwei oder mehreren Schritten vollzogen werden. Insbesondere sind Verfahren bevorzugt, in denen das acylierte Amin XII durch elektrophile Reagenzien in An- oder Abwesenheit von inerten Lösungsmitteln bei Temperaturen von -10°C bis 120°C, vorzugsweise von 0°C bis 100°C, in aromatische Sulfonsäuren sowie deren Derivate, zum Beispiel in Sulfonsäurehalogenide, überführt wird. Beispielsweise können Sulfonierungen mit Schwefelsäuren oder Oleum, Halogensulfonierungen mit Halogensulfonsäuren, Reaktionen mit Sulfurylhalogeniden in Gegenwart von wasserfreien Metallhalogeniden oder Thionylhalogeniden in Gegenwart von wasserfreien Metallhalogeniden mit anschließenden, in bekannter Weise durchgeführten, Oxidationen zu aromatischen Sulfonsäurechloriden, durchgeführt werden. Sind Sulfonsäuren die primären Reaktionsprodukte, so können diese entweder direkt oder durch Behandlung mit tertiären Aminen, wie Pyridin oder Trialkylaminen, oder mit Alkali- oder Erdalkalihydroxiden oder Reagenzien, welche diese basischen Verbindung in situ bilden, in bekannter Weise durch Säurehalogenide, wie Phosphortrihalogenide, Phosphorpentahalogenide, Phosphoroxychloride, Thionylhalogenide, Oxalylhalogenide, in Sulfonsäurehalogenide überführt werden. Die Überführung der Sulfonsäurederivate in Sulfonamide erfolgt in literaturbekannter Weise, vorzugsweise werden Sulfonsäurechloride in inerten Lösungsmitteln bei Temperaturen von 0°C bis 100°C mit wäßrigem Ammoniak in umgesetzt. Weiterhin kann man aromatische Sulfonamide nach literaturbeschriebenen Verfahren aus den nach Schema 1 hergestellten acylierten Aminen der Formel XII durch Umsetzungen mit alkali- oder erdalkalimetallorganischen Reagenzien in inerten Lösungsmitteln und unter Inertgasatmosphäre bei Temperaturen von -100°C bis 50°C, vorzugsweise von -100°C bis 30°C, mit Schwefeldioxid und anschließender thermischer Behandlung mit Amidosulfonsäure synthetisieren.

Je nach der Stellung des Substituenten R(1) in den acylierten Aminen XII kann die Einführung der Sulfamoylgruppe auch in eine der anderen Positionen des Thiophenringes verlaufen, beispielsweise in die 4-Position, wenn die 5-Position bereits durch R(1) besetzt ist.
Die erfindungsgemäßen Verbindungen 1 und ihre physiologisch unbedenklichen Salze können zur Herstellung pharmazeutischer Zubereitungen verwendet werden, insbesondere auf nichtchemischen Weg. Hierbei können sie zusammen mit mindestens einem festen, flüssigen Träger oder Hilfsstoff allein oder in Kombination mit anderen Herz-Kreislauf-aktiven Arzneimitteln, wie etwa Calcium-Antagonisten, NO-Donatoren oder ACE-Hemmern in eine geeignete Dosierungsform gebracht werden. Diese Zubereitungen können als Arzneimittel in der Human- oder Veterinärmedizin verwendet werden.
Als Trägerstoffe kommen organische oder anorganische Substanzen in Frage, die sich für die enterale (zum Beispiel orale), parenterale, zum Beispiel die intravenöse Applikation, oder topische Anwendungen eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Lösungen, vorzugsweise ölige oder wäßrige Lösungen, ferner Suspensionen, Emulsionen oder Implantate, für die topische Anwendung Salben, Cremes, Pasten, Lotionen, Gele, Sprays, Schäume, Aerosole, Lösungen (zum Beispiel in Alkoholen wie Ethanol oder Isopropanol, Acetonitril, DMF, Dimethylacetamid, 1,2-Propandiol oder deren Gemische untereinander oder mit Wasser) oder Puder. Die Verbindungen I können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht, die Stabilisierungs- und/oder Netzmittel, Emulgatoren, Salze und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und Geschmacks- und/oder Aromastoffe enthalten. Sie können falls erwünscht auch einen oder mehrere weitere Wirkstoffe enthalten, zum Beispiel ein oder mehrere Vitamine.
Die Dosierungen, die zur Behandlung von Herzrhythmusstörungen mit den Verbindungen I notwendig sind, hängen davon ab, ob akut oder prophylaktisch therapiert wird. Normalerweise kommt man mit einem Dosisbereich von etwa mindestens 0.1 mg, vorzugsweise mindestens 1 mg, bis höchstens 100, vorzugsweise bis höchstens 10 mg pro kg und Tag aus, bezogen auf einen Erwachsenen von 75 kg Gewicht, wenn Prophylaxe betrieben wird. Die Dosis kann dabei als orale oder parenterale Einzeldosis oder in bis zu vier Einzeldosen aufgeteilt werden. Werden akute Fälle von Herzrhythmusstörungen behandelt, beispielsweise auf einer Intensivstation, kann die parenterale Verabreichung vorteilhaft sein. Eine bevorzugte Dosisbereich in kritischen Situationen kann dann 10 bis 100 mg betragen und beispielsweise als intravenöse Dauerinfusion verabreicht werden.
Als Versuchstiere zum Nachweis solcher Wirkungen am Herzen eignen sich zum Beispiel Mäuse, Ratten, Meerschweinchen, Kaninchen, Hunde, Affen oder Schweine. Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Erfindungsgemäß können außer den in den Ausführungsbeispielen beschriebenen Verbindungen auch die in der folgenden Tabelle zusammengestellten Verbindungen I erhalten werden:
1) 2-[2-(5-Fluor-2-methoxybenzoylamino)ethyl]-5-(aminocarbonylaminosulfonyl)-thiophen
2) 2-[2-(5-Fluor-2-ethoxybenzoylamino)ethyl]-5-(aminocarbonylaminosulfonyl)-thiophen
3) 2-[2-(5-Chlor-2-methoxybenzoylamino)ethyl]-5-(methylaminocarbonylaminosulfonyl)-4-methylthiophen
4) 2-[2-(5-Chlor-2-methoxybenzoylamino)ethyl]-5-(aminocarbonylaminosulfonyl)-4-methylthiophen
5) 2-[2-(5-Fluor-2-methoxybenzoylamino)ethyl]-5-(aminothiocarbonylaminosulfonyl)-thiophen
6) 2-[2-(5-Fluor-2-ethoxybenzoylamino)ethyl]-5-(aminothiocarbonylaminosulfonyl)-thiophen
7) 2-[2-(5-Chlor-2-methoxybenzoylamino)ethyl]-5-(methylaminothiocarbonylaminosulfonyl)-4-methylthiophen
8) 2-[2-(5-Chlor-2-methoxybenzoylamino)ethyl]-5-(aminothiocarbonylaminosulfonyl)-4-methylthiophen

### Beispiele

### Beispiel 1

### 2-[2-(5-Chlor-2-methoxybenzoylamino)ethyl]-5-(methylaminocarbonylaminosulfonyl)-thiophen

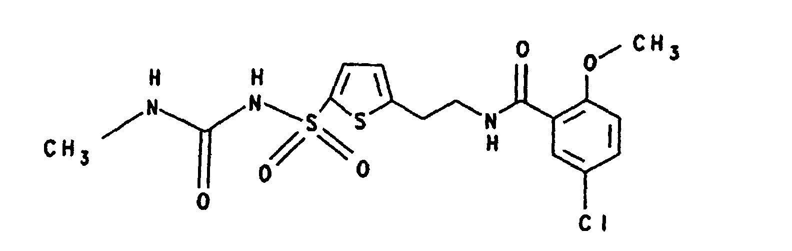

Zu 299 mg (0.8 mMol) 2-[2-(5-Chlor-2-methoxybenzoylamino)ethyl]-5-sulfamoylthiophen in 9 ml DMSO gibt man 120 mg pulverisiertes NaOH sowie 141 mg N-Methyltrichloracetamid (0.8 mMol). Die Mischung wird 1,5 Stunden bei 65°C gerührt. Nach dem Abkühlen wird in Eiswasser von pH 2 eingerührt. Der Niederschlag wird abgesaugt, in CH₂Cl₂ aufgenommen, mit Wasser gewaschen und mit MgSO₄ getrocknet. Nach dem Abdampfen des Lösemittels verbleibt ein gelbbraunes Rohprodukt das nach Behandlung mit CH₂Cl₂/5% Methanol bei -4°C kristallisiert. Man erhält 165 mg reines Produkt vom Schmp.: 160 - 161°C.

### Beispiel 2

### 2-[2-(5-Chlor-2-methoxybenzoylamino)ethyl]-5-(aminocarbonylaminosulfonyl)-thiophen

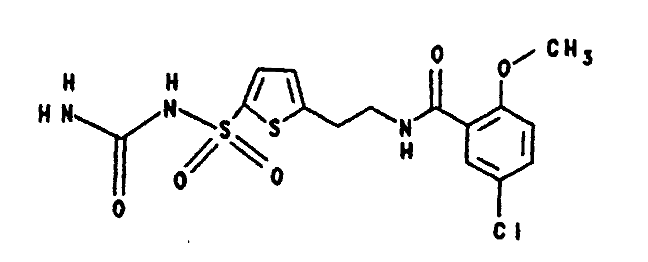

299 mg (0.8 mMol) 2-[2-(5-Chlor-2-methoxybenzoylaminolethyl]-5-sulfamoylthiophen werden mit 448 mg KOCN sowie 0.6 ml Triethylamin versetzt, und dann wird 4 h am Rückfluß gekocht. Die erkaltete Lösung wird filtriert und bis zur Trockene eingedampft. Der Rückstand wird mit 40 ml H₂O, 25 ml Triethylamin sowie 25 ml Essigsäureethylester gerührt. Die organische Phase wird 3 mal mit Wasser/1%-Triethylamin extrahiert. Die vereinigten wäßrigen Phasen werden mit 2N Salzsäure angesäuert. Der sich bildende Niederschlag wird 3 Stunden im Eisbad gerührt und abgesaugt. Schmp.: 172 - 174°C.

### Beispiel 3

### 2-[2-(5-Chlor-2-methoxybenzoylamino)ethyl]-3 methyl 5-(methylaminocarbonylaminosulfonyl)-thiophen

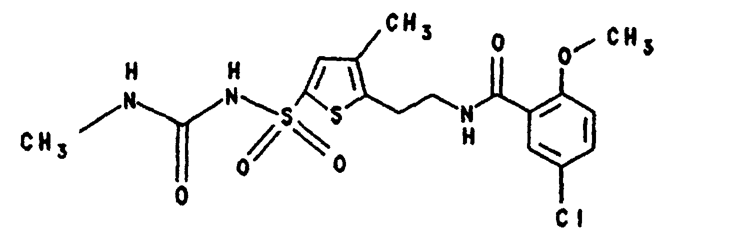

Herstellung wie in Beispiel 1 aus 2-[2-(5-Chlor-2-methoxybenzoylamino)ethyll-3-methyl-5-sulfamoyl-thiophen und Trichloracetamid. Das Rohprodukt wird durch Rühren in 30°C warmen Essigester gereinigt.
Schmp.: 185 - 187°C

### Beispiel 4

### 2-[2-(5-Chlor-2-methoxybenzoylamino)ethyl]-3 methyl-5-(aminocarbonylaminosulfonyl)-thiophen

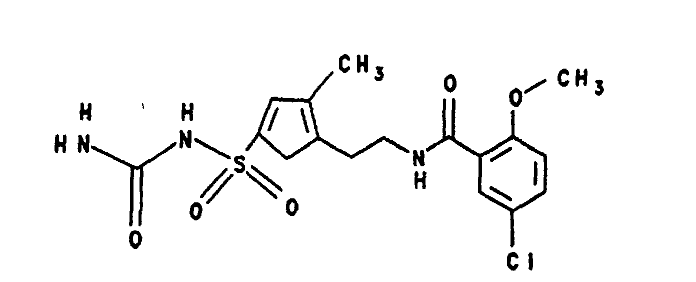

311 mg (0.8 mMol) 2-[2-(5-Chlor-2-methoxybenzoylamino)ethyl]-3-methyl-5-sulfamoyl-thiophen werden mit 448 mg KOCN sowie 0.6 ml Triethylamin versetzt, und dann wird 4 h am Rückfluß gekocht. Die erkaltete Lösung wird filtriert und bis zur Trockene eingedampft. Der Rückstand wird mit 40 ml H₂O, 25 ml Triethylamin sowie 25 ml Essigsäureethylester gerührt. Die organische Phase wird 3 mal mit Wasser/1% Triethylamin extrahiert. Die vereinigten wäßrigen Phasen werden mit 2N Salzsäure angesäuert. Der sich bildende Niederschlag wird 3 Stunden im Eisbad gerührt und abgesaugt.
Schmp.: 174 - 176°C.

### Beispiel 5

### 2-[2-(5-Chlor-2-methoxybenzoylamino)ethyl]-5-(mathylaminothiocarbonylaminosulfonyl)-thiophen

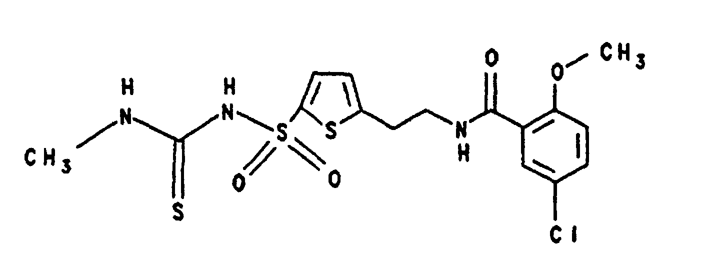

375 mg 2-[2-(5-Chlor-2-methoxybenzoylamino)ethyl]-5-sulfamoyl-thiophen werden zusammen mit 415 mg K₂CO₃ und 90 mg Methylisothiocyanat in 3 ml absolutem DMF gelöst, und dann wird 3 h bei 60°C gerührt. Man trägt die erkaltete Lösung in 2N HCl ein und saugt den entstandenen Niederschlag ab. Der getrocknete Rückstand wird an Kieselgel mit dem Laufmittelsystem Essigsäureethylacetat/Toluol 1 : 1 chromatographiert.
Ausbeute: 228 mg mit Schmp.: 182 - 184°C.

### Beispiel 6

### 2-[2-(5-Chlor-2-methoxybenzoylamino)ethyl)-4-(methylarmnocarbonylaminosulfonyl)-5-methyl-thiophen

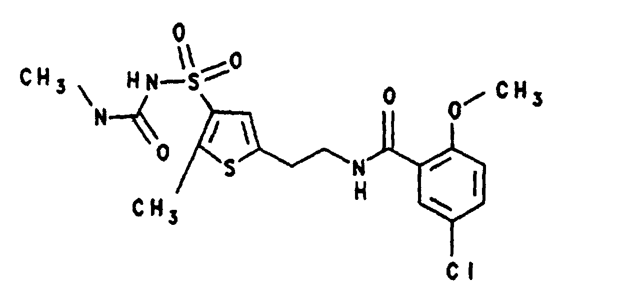

Analog Beispiel 1 aus 2-[2-(5-Chlor-2-methoxybenzoylamino)ethyl]-5-methyl-4-sulfamoyl-thiophen und Trichloracetamid.
Schmp.: 198 - 199°C

### Beispiel 7

### 2-[2-(5-Chlor-2-methoxybenzoylamino)ethyl]-4-(aminocarbonylaminosulfonyl)-5-methyl-thiophen

Analog Beispiel 2 aus 2-[2-(5-Chlor-2-methoxybenzoylamino)ethyl]-4-sulfamoyl-5-methyl-thiophen und KOCN.
Schmp.: 160- 161°C.

**Pharmakologische Daten:**

Mit den folgenden Modellen können die therapeutischen Eigenschaften der Verbindungen I nahegelegt werden:

### (1) Aktionspotentialdauer am Papillarmuskel des Meerschweinchens:

### (a) Einleitung

ATP-Mangelzustände wie sie während einer Ischämie in der Herzmuskelzelle beobachtet werden führen zu einer Verkürzung der Aktionspotentialdauer. Sie gelten als einer der Ursachen für sogenannte Reentry-Arrhythmien die den plötzlichen Herztod verursachen können. Die Öffnung von ATP-sensitiven K-Kanälen durch das Absinken von ATP gilt hierfür als ursächlich.

### (b) Methode

Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9% NaCI, 0.048% KCl, 0.024% CaCl₂, 0.02% NaHCO₃ und 0.1% Glucose) durchspült und mit einer Mischung aus 95% Sauerstoff und 5% Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 mMol KCI-Lösung gefüllt ist, abgeleitet und registriert. Die zu prüfenden Substanzen wurden der Ringerlösung in einer Konzentration von 2.2·10⁻⁵ Mol pro Liter zugesetzt. Das Aktionspotential wird mit einem Amplifier von Hugo Sachs verstärkt und am Oszilloskop dargestellt. Die Dauer des Aktionspotentials wird bei einem Repolarisierungsgrad von 95% (APD95) bestimmt. Aktionspotentialverkürzungen werden entweder durch Zugabe einer 1 µM starken Lösung des Kaliumkanalöffners Hoe 234 (J. Kaiser, H. Gögelein, Naunyn-Schmiedebergs Arch. Pharm. 1991, 343, R 59)oder durch Zugabe von 2-Desoxyglucose hervorgerufen. Der Aktionspotential-verkürzende Effekt dieser Substanzen wurde durch die gleichzeitige Gabe der Testsubstanzen verhindert oder verringert. Testsubstanzen wurden als Stammlösungen in Propandiol der Badlösung zugesetzt. Die angegebenen Werte beziehen sich auf Messungen 30 min nach der Zugabe. Glibenclamid diente in diesen Messungen als Standard. Die Testkonzentration beträgt in allen Fällen 2 x 10⁻⁵ M

### (c) Resultate:

| Beispiel-Nr | APD95-Anfang [ms] | APD95-30 min [ms] |
|---|---|---|
| 1 | 168 ± 15 | 150 ± 13 |
| 2 | 193 | 131 |

### (2) Membranpotential an isolierten β-Zellen:

### (a) Einleitung

Der Wirkmechanismus der blutzuckersenkenden Sulfonylharnstoffe ist in groben Zügen aufgeklärt. Zielorgan sind die β-Zellen des Pankreas, wo es zu einer Mehrausschüttung des Blutzucker-senkenden Hormons Insulin kommt. Die Freisetzung von Insulin wird über das Zellmembranpotential gesteuert. Glibenclamid bewirkt eine Depolarisation der Zellmembran was über einen vermehrten Einstrom von Calciumionen die Insulinfreisetzung fördert. Das Ausmaß dieser Depolarisation der Zellmembran ΔU wurde an RINm5F Zellen, einer Pankreastumorzellinie, für einige der erfindungsgemäßen Verbindungen bestimmt. Die Wirkstärke einer Verbindung in diesem Modell sagt das Ausmaß des blutzuckersenkenden Potentials dieser Verbindung voraus.

### (b) Methode

### Zellkultur von RINm5F Zellen

RINm5F Zellen wurden in RPMI 1640 Kulturmedium (Flow), dem 11 mM Glukose, 10% (vol/vol) fötales Kälberserum, 2 mM Glutamin und 50 µg/ml Gentamycin zugesetzt wurde, bei 37°C kultiviert. Für die Untersuchungen wurden die Zellen durch Inkubation (ca. 3 min) in einem Ca²⁺-freien Medium, das 0.25% Trypsin enthielt, isoliert und auf Eis aufbewahrt.

### Meßmethode

Isolierte RINm5F Zellen wurden in eine Plexiglaskammer auf einem inversen Mikroskop, das mit Differential-Interferenz-Kontrast Optik ausgerüstet ist, gebracht. Unter optischer Kontrolle (400-fache Vergrößerung) wurde eine feuerpolierte Mikropipette mit Öffungsdurchmessser von etwa 1 pm mit Hilfe eines Mikromanipulators auf die Zelle aufgesetzt. Durch Anlegen eines leichten Unterdruckes in der Patch-Pipette wurde zunächst eine hohe elektrische Abdichtung zwischen Glas und Zellmembran hergestellt und anschließend durch Erhöhung des Unterdruckes der Membranfleck unter der Meßpipette aufgerissen. In dieser Ganzzellenkonfiguration wurde mit Hilfe eines Patch-Clamp Verstärkers (L/M EPC 7) das Zellpotential registriert und durch Anlegen einer Spannungsrampe der Ganzzellenstrom gemessen. Lösungen: Die Patch-Pipette war mit KCI-Lösung gefüllt (in mMol pro Liter): 140 KCI, 10 NaCl, 1.1 MgCl₂, 0.5 EGTA, 1 Mg-ATP, 10 HEPES, pH = 7.2 und im Bad befand sich NaCl-Lösung (in mMol pro Liter): 140 NaCl, 4.7 KCl, 1.1 MgCl₂, 2 CaCl₂, 10 HEPES, pH = 7.4. Von den Testsubstanzen wurden Stocklösungen (Konzentration 100 mmol) in Dimethylsulfoxid (DMSO) und entsprechende Verdünnungen in NaCl-Lösung hergestellt. DMSO alleine hatte keinen Effekt auf das Zellpotential. Um das Zellpotential unter Kontrollbedingungen zu stabilisieren, wurde der Öffner für ATP-sensitive K⁺-Kanäle Diazoxid (100 µmol) bei allen Versuchen in die Badlösung zugegeben. Alle Experimente wurden bei 34 ± 1°C durchgeführt.

### (c) Ergebnisse (Die Konzentrationen der erfindungsmäßigen Verbindungen betragen in den Versuchen 10⁻⁵ Mol pro Liter)

| Beispiel-Nr | ΔU (mV) |
|---|---|
| 1 | 5 (Leerwert: -80 mV) |
| 2 | 4 (Leerwert: -79mV) |

## Patentansprüche

1. Substituierte Thiophensulfonylharnstoffe und -thioharnstoffe der Formel I, 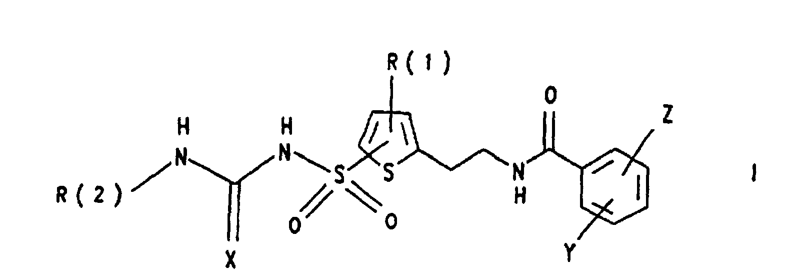 in der bedeuten:
R(1) Wasserstoff oder Methyl;
R(2) Wasserstoff oder Methyl;
X Sauerstoff oder Schwefel;
Z, Y voneinander verschieden, Fluor, Chlor, Methoxy oder Ethoxy;
und ihre physiologisch unbedenklichen Salze.

2. Verbindungen der Formel I nach Anspruch 1, in der bedeuten:
R(1) Wasserstoff oder Methyl;
R(2) Wasserstoff oder Methyl;
X Sauerstoff;
Z, Y voneinander verschieden, Fluor, Chlor, Methoxy oder Ethoxy;
und ihre physiologisch unbedenklichen Salze.

3. Verbindungen der Formel I nach Anspruch 1, in der bedeuten:
R(1) Wasserstoff oder Methyl;
R(2) Wasserstoff oder Methyl;
X Schwefel;
Z, Y voneinander verschieden, Fluor, Chlor, Methoxy oder Ethoxy,
und ihre physiologisch unbedenklichen Salze.

4. Verfahren zur Herstellung von einer Verbindung der Formel I nach einem oder mehreren der Ansprüche 1 bis 3, **dadurch gekennzeichnet, daß** man
(a) ein aromatisches Sulfonamid der Formel II 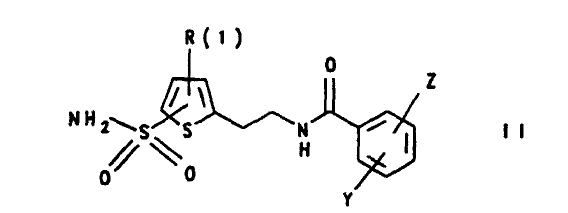 oder dessen Salz der Formel III, 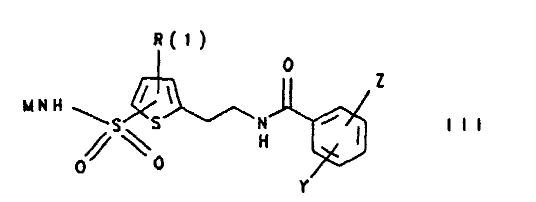 worin R(1), Y und Z die in den Ansprüchen 1 bis 3 angegebenen Bedeutungen haben und worin M für ein Alkali-, Erdalkali-, Ammonium- oder Tetraalkylammonium-lon steht, mit einem R(2)-substituierten Isocyanat der Formel IV,
R(2)-N=C=O IV
worin R(2) die in den Ansprüchen 1 bis 3 angegebene Bedeutung hat, zu einem substituierten Thiophensulfonylharnstoff der Formel la umsetzt; 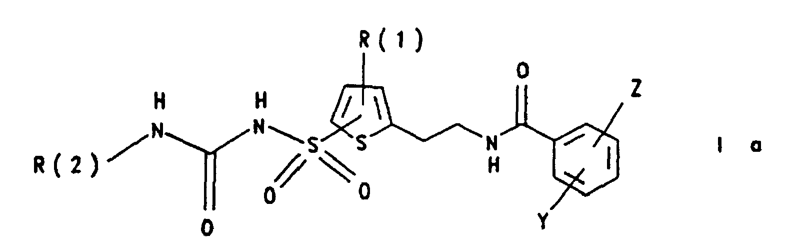 oder
(b) einen unsubstituierten Thiophensulfonylharnstoff der Formel la [R(2) = H] durch Umsetzung eines aromatischen Thiophensulfonamids der Formel II oder von dessen Salz der Formel III mit einem Trialkylsilylisocyanat oder Siliciumtetraisocyanat und Spaltung des primären siliciumsubstituierten Thiophensulfonylharnstoffs herstellt;
oder
(c) einen Thiophensulfonylharnstoff der Formel la aus einem aromatischen Thiophensulfonamid der Formel II oder dessen Salz der Formel III durch Umsetzung mit einem R(2)-substituierten Trichloracetamid der Formel V 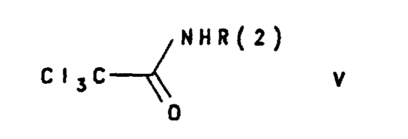 in Gegenwart einer Base herstellt;
oder
(d) einen Thiophensulfonylthioharnstoff der Formel Ib 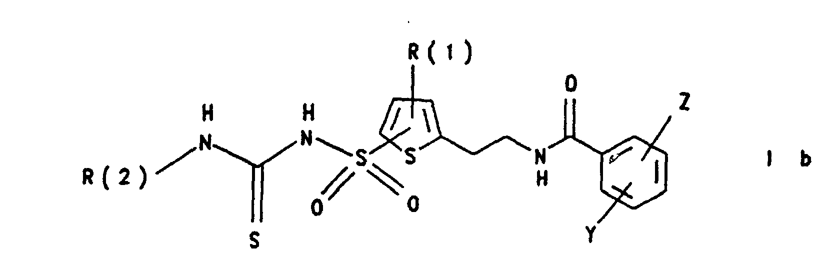 aus einem Thiophensulfonamid der Formel II oder dessen Salz der Formel III durch Umsetzung mit einem R(2)-substituierten Isothiocyanat der Formel VI
R(2)-N=C=S VI
herstellt;
oder
(e) einen substituierten Thiophensulfonylharnstoff der Formel la durch Umwandlung eines Thiophensulfonylthioharnstoffs der Formel Ib herstellt; oder
(f) einen Thiophensulfonylharnstoff der Formel la aus einem Thiophensulfonylhalogenid der Formel VII 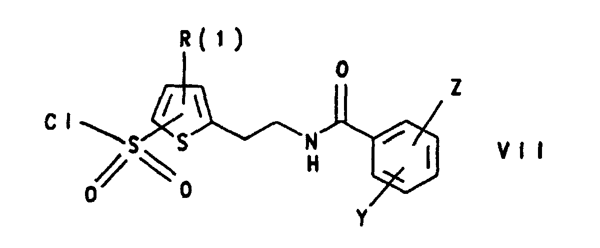 mit einem R(2)-substituierten Harnstoff oder R(2)-substituierten Bis(trialkylsilyl)harnstoff herstellt;
oder
(g) einen Thiophensulfonylharnstoff der Formel la durch Umsetzung eines Amins der Formel R(2)-NH₂ mit einem Thiophensulfonylisocyant der Formel VIII 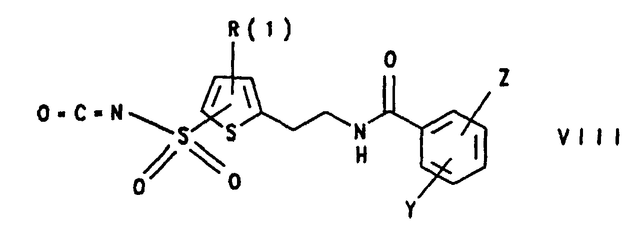 herstellt;
oder
(h) einen Thiophensulfonylthioharnstoff der Formel Ib durch Umsetzung eines Amins der Formel R(2)-NH₂ mit einem Thiophensulfonylisothiocyanat der Formel IX 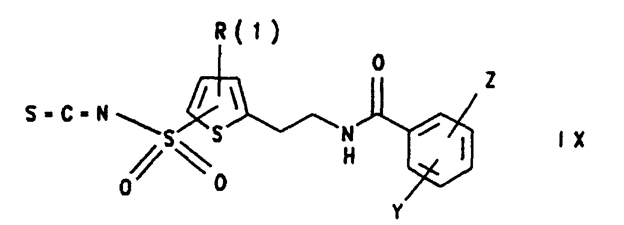 herstellt;
oder
(i) einen Benzolsulfenyl- oder -sulfinylharnstoff zu einem Thiophensulfonylharnstoff der Formel la oxidiert.

5. Verwendung einer Verbindung der Formel I nach einem oder mehreren der Ansprüche 1 bis 3 und/oder ihrer physiologisch unbedenklichen Salze zur Herstellung eines Medikaments zur Behandlung von Herzrhythmusstörungen.

6. Verwendung einer Verbindung der Formel I nach einem oder mehreren der Ansprüche 1 bis 3 und/oder ihrer physiologisch unbedenklichen Salze zur Herstellung eines Medikaments zur Verhinderung des plötzlichen Herztods.

7. Verwendung einer Verbindung der Formel I nach einem oder mehreren der Ansprüche 1 bis 3 und/oder ihrer physiologisch unbedenklichen Salze zur Herstellung eines Medikaments zur Behandlung von ischämischen Zuständen des Herzens.

8. Verwendung einer Verbindung der Formel I nach einem oder mehreren der Ansprüche 1 bis 3 und/oder ihrer physiologisch unbedenklichen Salze zur Herstellung eines wissenschaftliches Tools zur Inhibition ATP-sensitiver Kaliumkanäte

9. Verwendung einer Verbindung der Formel I nach einem oder mehreren der Ansprüche 1 bis 3 und/oder ihrer physiologisch unbedenklichen Salze zur Herstellung eines Medikaments zur Behandlung der geschwächten Herzkraft.

10. Verwendung einer Verbindung der Formel I nach einem oder mehreren der Ansprüche 1 bis 3 und/oder ihrer physiologisch unbedenklichen Salze zur Herstellung eines Medikaments zur Verbesserung der Herzfunktion nach Herztransplantation.

11. Heilmittel, **gekennzeichnet durch** eine wirksame Menge an einer Verbindung der Formel I nach einem oder mehreren der Ansprüche 1 bis 3 und/oder ihrer physiologisch unbedenklichen Salze.

## Claims

1. A substituted thiophenesulfonylurea or -thiourea of the formula I 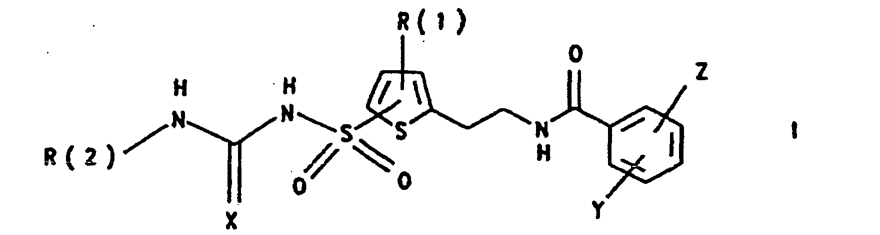 in which:
R(1) is hydrogen or methyl;
R(2) is hydrogen or methyl;
X is oxygen or sulfur;
Z and Y are different from one another and are fluorine, chlorine, methoxy or ethoxy;
or a physiologically acceptable salt thereof.

2. A compound of the formula I as claimed in claim 1, in which:
R(1) is hydrogen or methyl;
R(2) is hydrogen or methyl;
X is oxygen;
Z and Y are different from one another and are fluorine, chlorine, methoxy or ethoxy;
or a physiologically acceptable salt thereof.

3. A compound of the formula I as claimed in claim 1, in which:
R(1) is hydrogen or methyl;
R(2) is hydrogen or methyl;
X is sulfur;
Z and Y are different from one another and are fluorine, chlorine, methoxy or ethoxy,
or a physiologically acceptable salt thereof.

4. A process for the preparation of a compound of the formula I as claimed in one or more of claims 1 to 3, which comprises
(a) reacting an aromatic sulfonamide of the formula II 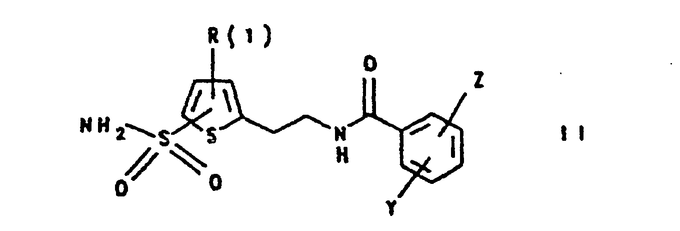 or its salt of the formula III 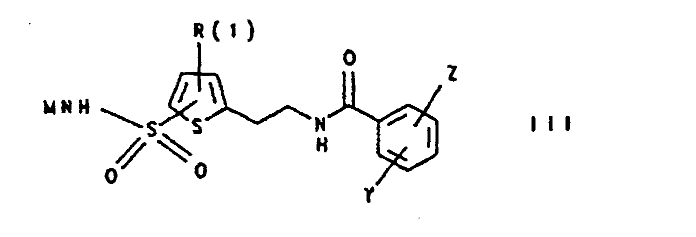 in which R(1), Y and Z have the meanings indicated in claims 1 to 3 and in which M is an alkali metal, alkaline earth metal, ammonium or tetraalkylammonium ion,
with an R(2)-substituted isocyanate of the formula IV
R(2)-N=C=O IV
in which R(2) has the meaning indicated in claims 1 to 3,
to give a substituted thiophenesulfonylurea of the formula Ia; 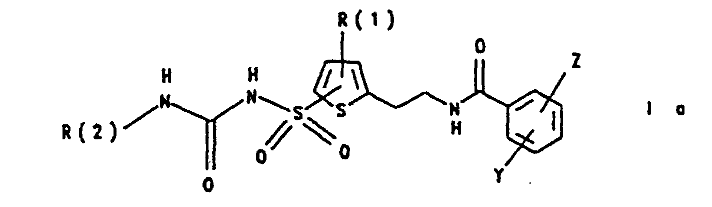 or
(b) preparing an unsubstituted thiophenesulfonylurea of the formula Ia [R(2) = H] by reaction of an aromatic thiophenesulfonamide of the formula II or of its salt of the formula III with a trialkylsilyl isocyanate or silicon tetraisocyanate and cleavage of the primary silicon-substituted thiophenesulfonylurea;
or
(c) preparing a thiophenesulfonylurea of the formula Ia from an aromatic thiophenesulfonamide of the formula II or its salt of the formula III by reaction with an R(2)-substituted trichloroacetamide of the formula V 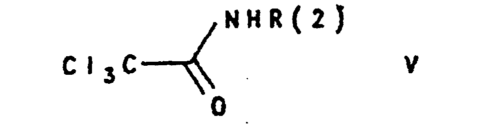 in the presence of a base;
or
(d) preparing a thiophenesulfonylthiourea of the formula Ib 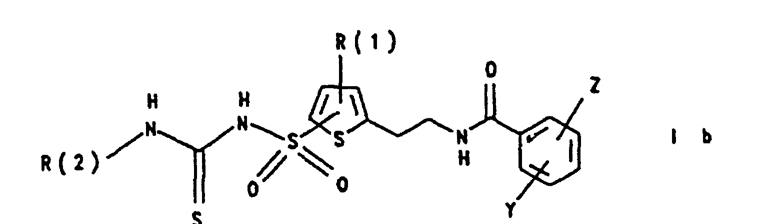 from a thiophenesulfonamide of the formula II or its salt of the formula III by reaction with an R(2)-substituted isothiocyanate of the formula VI
R(2)-N=C=S VI;
or
(e) preparing a substituted thiophenesulfonylurea of the formula Ia by conversion of a thiophenesulfonylthiourea of the formula Ib;
or
(f) preparing a thiophenesulfonylurea of the formula Ia from a thiophenesulfonyl halide of the formula VII 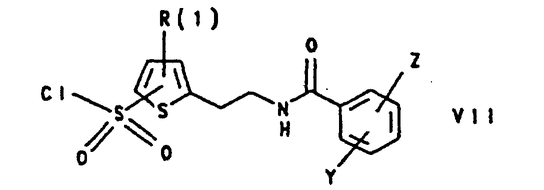 using an R(2)-substituted urea or R(2)-substituted bis(trialkylsilyl)urea;
or
(g) preparing a thiophenesulfonylurea of the formula Ia by reaction of an amine of the formula R(2)-NH₂ with a thiophenesulfonyl isocyanate of the formula VIII 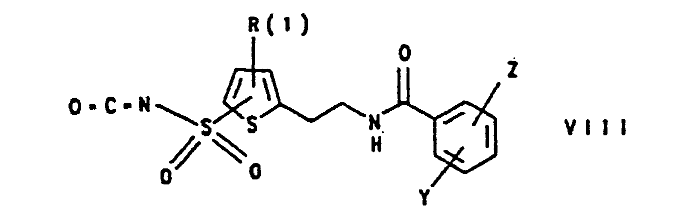 or
(h) preparing a thiophenesulfonylthiourea of the formula Ib by reaction of an amine of the formula R(2)-NH₂ with a thiophenesulfonyl isothiocyanate of the formula IX 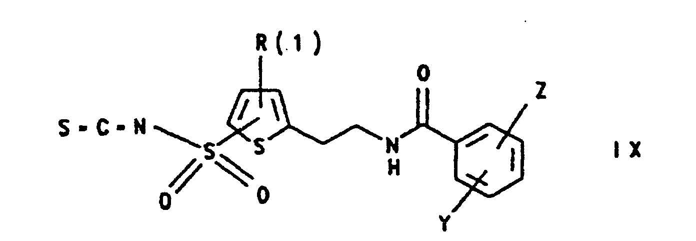 or
(i) oxidizing a benzenesulfenyl- or -sulfinylurea to give a thiophenesulfonylurea of the formula Ia.

5. The use of a compound of the formula I as claimed in one or more of claims 1 to 3 and/or a physiologically acceptable salt thereof for the production of a medicament for the treatment of cardiac arrhythmias.

6. The use of a compound of the formula I as claimed in one or more of claims 1 to 3 and/or a physiologically acceptable salt thereof for the production of a medicament for the prevention of sudden heart death.

7. The use of a compound of the formula I as claimed in one or more of claims 1 to 3 and/or a physiologically acceptable salt thereof for the production of a medicament for the treatment of ischemic conditions of the heart.

8. The use of a compound of the formula I as claimed in one or more of claims 1 to 3 and/or a physiologically acceptable salt thereof for the production of a scientific tool for the inhibition of ATP-sensitive potassium channels.

9. The use of a compound of the formula I as claimed in one or more of claims 1 to 3 and/or its physiologically acceptable salts for the production of a medicament for the treatment of weakened cardiac power.

10. The use of a compound of the formula I as claimed in one or more of claims 1 to 3 and/or its physiologically acceptable salts for the production of a medicament for the improvement of heart function after heart transplantation.

11. A medicament comprising an effective amount of a compound of the formula I as claimed in one or more of claims 1 to 3 and/or a physiologically acceptable salt thereof.

## Revendications

1. Thiophènesulfonylurées et thiophènesulfonylthiourées substituées de formule I 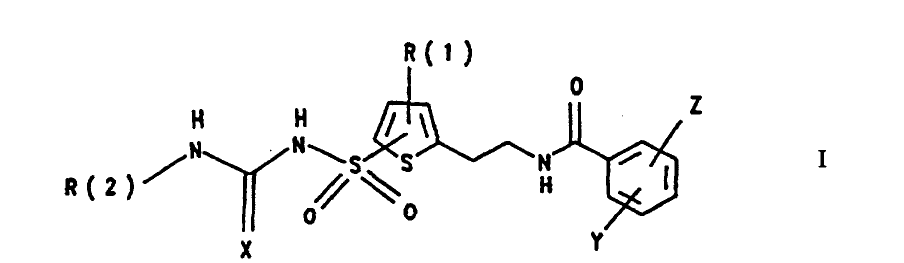 dans laquelle
R(1) représente un atome d'hydrogène ou un reste méthyle;
R(2) représente un atome d'hydrogène ou un reste méthyle;
X représente un atome d'oxygène ou de soufre;
Z, Y, différents l'un de l'autre, représentent un reste fluoro, chloro, méthoxy ou éthoxy:
et leurs sels physiologiquement acceptables.

2. Composés de formule I selon la revendication 1, dans lesquels:
R(1) représente un atome d'hydrogène ou un reste méthyle;
R(2) représente un atome d'hydrogène ou un reste méthyle;
X représente un atome d'oxygène;
Z, Y, différents l'un de l'autre, représentent un reste fluoro, chloro, méthoxy ou éthoxy;
et leurs sels physiologiquement acceptables.

3. Composés de formule I selon la revendication 1, dans lesquels:
R(1) représente un atome d'hydrogène ou un reste méthyle;
R(2) représente un atome d'hydrogène ou un reste méthyle;
X représente un atome de soufre;
Z, Y, différents l'un de l'autre, représentent un reste fluoro, chloro, méthoxy ou éthoxy;
et leurs sels physiologiquement acceptables.

4. Procédé de préparation d'un composé de formule I selon l'une ou plusieurs des revendications 1 à 3, **caractérisé en ce que**
(a) on fait réagir un sulfonamide aromatique de formule II 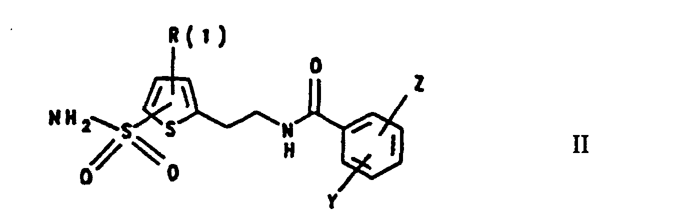 ou son sel de formule III 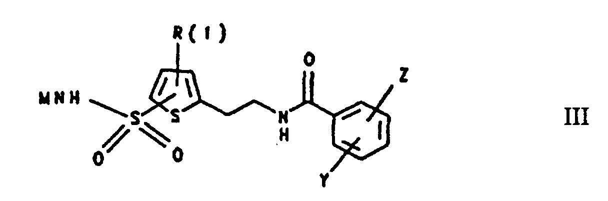 où R(1), Y et Z ont la signification indiquée dans les revendications 1 à 3, et où M représente un ion de métal alcalin, de métal alcalino-terreux, d'ammonium ou de tétraalkylammonium, avec un isocyanate substitué par R(2) de formule IV
R(2)-N=C=O IV
dans laquelle R(2) a la signification indiquée dans les revendications 1 à 3, pour obtenir une thiophènesulfonylurée substituée de formule Ia 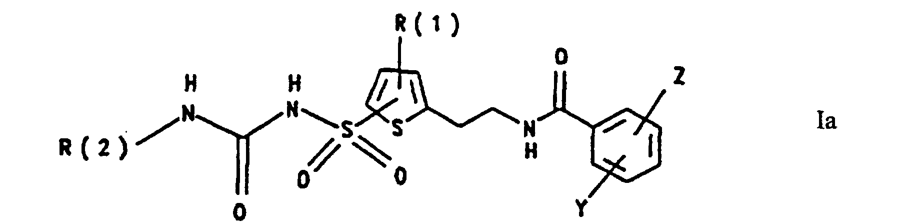 ou
(b) on prépare une thiophènesulfonylurée non substituée de formule Ia [R(2) = H] par réaction d'un thiophènesulfonamide aromatique de formule II ou de son sel de formule III avec un isocyanate de trialkylsilyle ou du tétraisocyanate de silicium et dissociation de la thiophènesulfonylurée substituée par du silicium primaire;
ou
(c) on prépare une thiophènesulfonylurée de formule Ia à partir d'un thiophènesulfonamide aromatique de formule II ou de son sel de formule III par réaction avec un trichloroacétamide substitué par R(2) de formule V 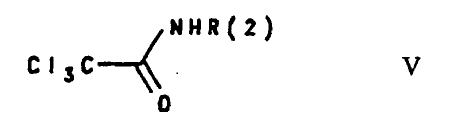 en présence d'une base;
ou
(d) on prépare une thiophènesulfonylthiourée de formule Ib 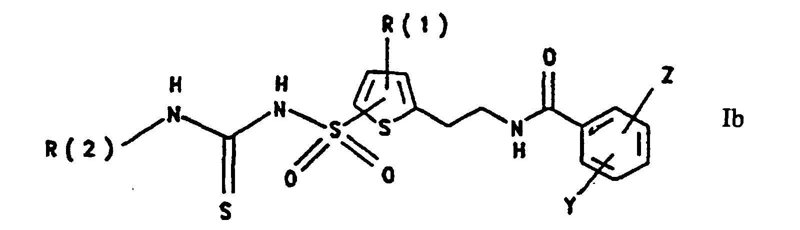 à partir d'un thiophènesulfonamide de formule II ou de son sel de formule III par réaction avec un isothiocyanate substitué par R(2) de formule VI
R(2)-N=C=S VI
ou
(e) on prépare une thiophènesulfonylurée substituée de formule Ia par transformation d'une thiophènesulfonylthiourée de formule Ib;
ou
(f) on prépare une thiophènesulfonylurée substituée de formule Ia à partir d'un halogénure de thiophènesulfonyle de formule VII 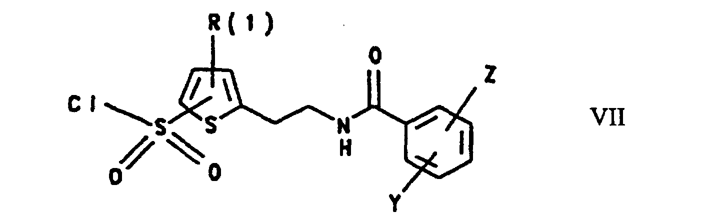 avec une urée substituée par R(2) ou une bis(trialkylsilyl)urée substituée par R(2);
ou
(g) on prépare une thiophènesulfonylurée de formule la par réaction d'une amine de formule R(2)-NH₂ avec un isocyanate de thiophènesulfonyle de formule VIII 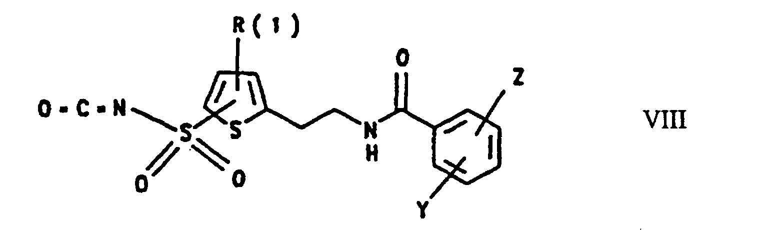 ou
(h) on prépare une thiophènesulfonylthiourée de formule Ib par réaction d'une amine de formule R(2)-NH₂ avec un isothiocyanate de thiophènesulfonyle de formule IX 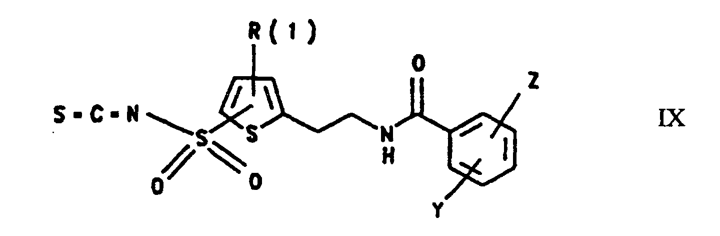 ou
(i) on oxyde une benzènesulfényl- ou -sulfinylurée en une thiophènesulfonylurée de formule Ia.

5. Utilisation d'un composé de formule I selon l'une ou plusieurs des revendications 1 à 3 et/ou de leurs sels physiologiquement acceptables pour la préparation d'un médicament destiné au traitement de troubles du rythme cardiaque.

6. Utilisation d'un composé de formule I selon l'une ou plusieurs des revendications 1 à 3 et/ou de leurs sels physiologiquement acceptables pour la préparation d'un médicament destiné à empêcher la mort brutale par arrêt cardiaque.

7. Utilisation d'un composé de formule I selon l'une ou plusieurs des revendications 1 à 3 et/ou de leurs sels physiologiquement acceptables pour la préparation d'un médicament destiné au traitement d'états ischémiques du coeur.

8. Utilisation d'un composé de formule I selon l'une ou plusieurs des revendications 1 à 3 et/ou de leurs sels physiologiquement acceptables pour la préparation d'un outil scientifique destiné à l'inhibition des canaux potassiques sensibles à l'ATP.

9. Utilisation d'un composé de formule I selon l'une ou plusieurs des revendications 1 à 3 et/ou de leurs sels physiologiquement acceptables pour la préparation d'un médicament destiné au traitement des insuffisances cardiaques.

10. Utilisation d'un composé de formule I selon l'une ou plusieurs des revendications 1 à 3 et/ou de leurs sels physiologiquement acceptables pour la préparation d'un médicament destiné à améliorer la fonction cardiaque après une greffe cardiaque.

11. Médicament **caractérisé en ce qu'**il contient une quantité active d'un composé de formule I selon l'une ou plusieurs des revendications 1 à 3 et/ou de ses sels physiologiquement acceptables.
